# EUROPEAN PATENT SPECIFICATION

(11) **EP 4 146 324 B1**
(45) Date of publication and mention of the grant of the patent: **01.07.2026**
(21) Application number: 21800598.1
(22) Date of filing: 08.05.2021
(51) Int. Cl.: A61M 39/16, A61M 39/20

(54) **DISINFECTION CAP FOR NEEDLE-FREE MEDICAL VALVES**
DESINFEKTIONSKAPPE FÜR NADELFREIE MEDIZINISCHE VENTILE
CAPUCHON DE DÉSINFECTION POUR VALVES MÉDICALES SANS AIGUILLE

(30) Priority: 08.05.2020 TR 202007203
(43) Date of publication of application: 15.03.2023
(73) Proprietor: Kürümoglu, Servet, Sincan/Ankara (TR)
(72) Inventor: GÜRSU, Hakan, Çankaya/Ankara (TR)
(86) International application number: PCT/TR2021/050449
(87) International publication number: WO 2021/225556

(56) References cited:
- WO-A2-2012/112815
- GB-A- 2 453 361
- US-A1- 2010 047 123
- US-A1- 2013 171 030
- US-A1- 2016 144 118
- US-A1- 2019 234 540
- US-A1- 2019 234 540

## Description

### Technical Field

The invention relates to operating methods of the disinfection caps used for the male or female connections of needle-free medical valves.

### State of the Art

As is known, the disinfection caps for the medical valves comprise one-type connections and are produced as two different products specialized for the female and/or male connections. Existing products' bodies are generally cylindrical and there are recesses and protrusions on the surface. Products vary in size, form and volume, and are disclosed exemplarily in US 2019/234540 A1, US 2010/047123 A1 and GB 2 453 361 A.

### Technical Problems to be Solved by the Invention

Existing systems allow one-type connections as a female or male connection. Therefore, different products are used for the two connection types. Using different products for the connections causes problems related to storing and ease of use. The objective of the invention is that a system presents in a body can be used for two different connections.

In the existing systems, the disinfectant holder member inside the cap can be compressed by attaching the cap to the female or male connections. Disinfectants are used such as alcohol-based etc. disinfectant solution, antibacterial solution, antifungal solution or isopropyl alcohol which is placed in the cap having low heat of evaporation for using as disinfectant. Losses are seen in distribution of the disinfectant solution, antibacterial solution, antifungal solution or isopropyl alcohol which is used in the caps on all surfaces of the female or male connection. Another objective of the invention is to allow the distribution of the solutions or isopropyl alcohol contained in the cap to all of the contacting surfaces. Due to locations of the slot, piston and disinfectant holder member, the solutions or isopropyl alcohol are distributed when the female or male connection enters the system.

Existing caps are generally in cylindrical shape and have mostly on the outer surface thereof recesses and protrusions in order to increase their grip and hold capability on their outer surfaces which triggers microbial contaminations. Another objective of the invention is to increase the grip and hold capability by its triangular form and to reduce the microbial contaminations by the flat surface it has.

### Description of the Drawings

In order to explain the cap developed with this invention better, the drawings and related explanations used are given below.

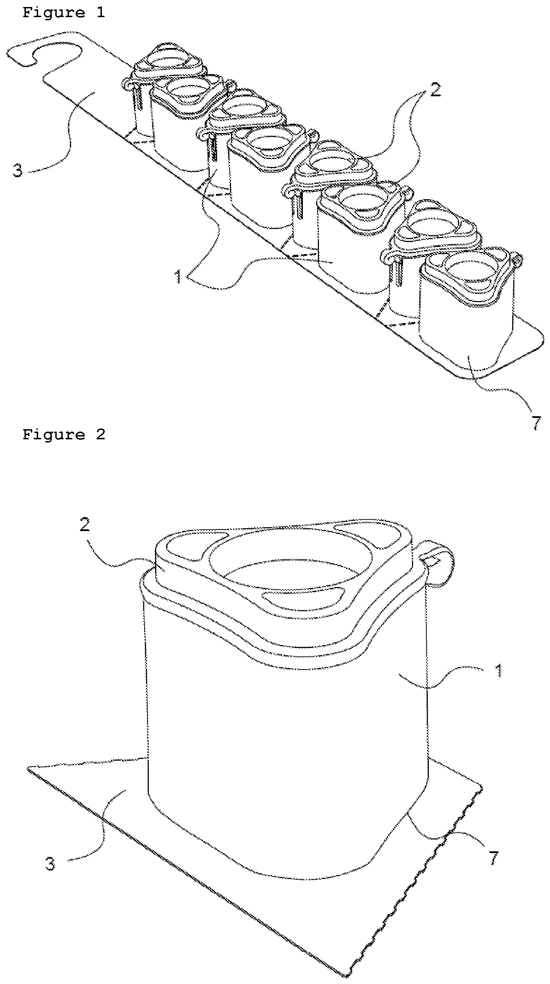

- Figure 1:: View of the product in sets,
- Figure 2:: Perspective view of the product with the caps being closed,

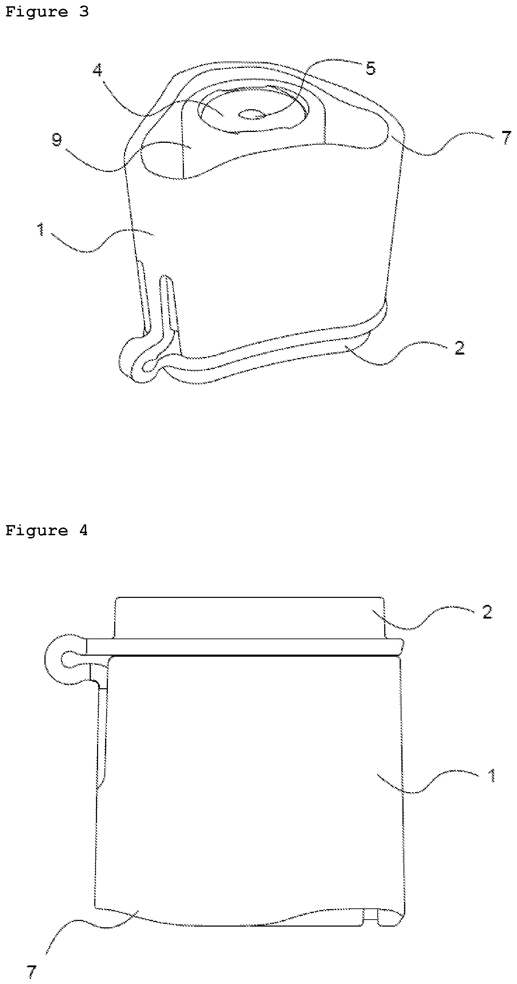

- Figure 3:: Perspective view of the product with the foil cap being open,
- Figure 4:: Front view of the product with the foil cap being open,

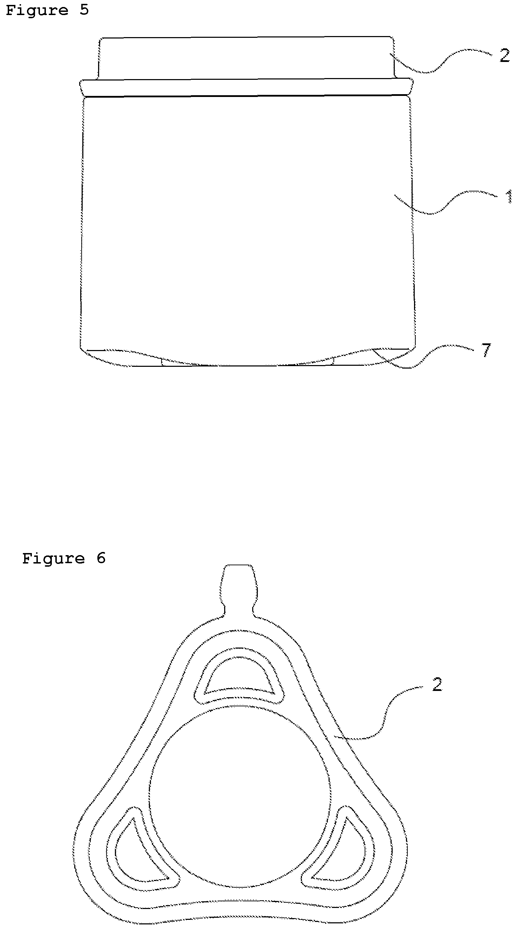

- Figure 5:: Side view of the product with the foil cap being open,
- Figure 6:: Top view of the product with the foil cap being open

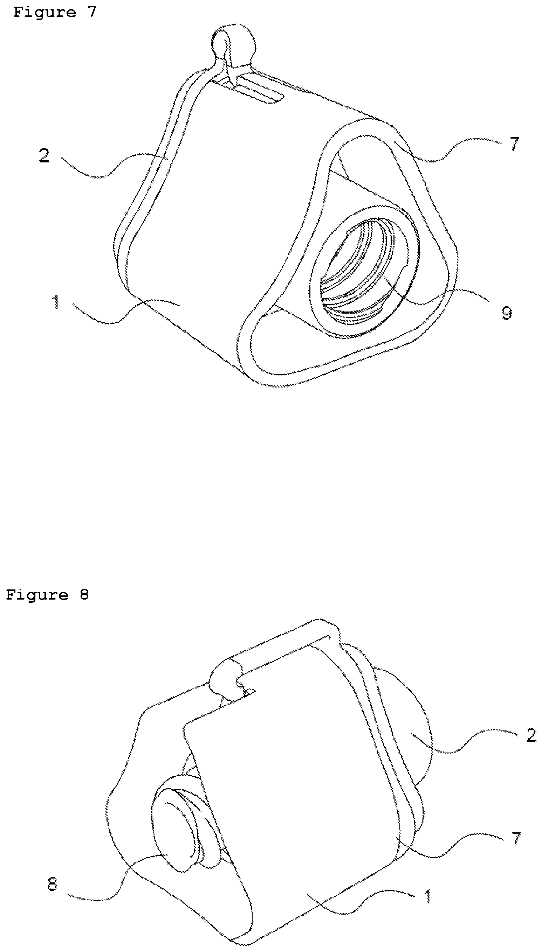

- Figure 7:: Perspective view of the product with the foil cap being open,
- Figure 8:: Perspective view of the product with the cap being open,

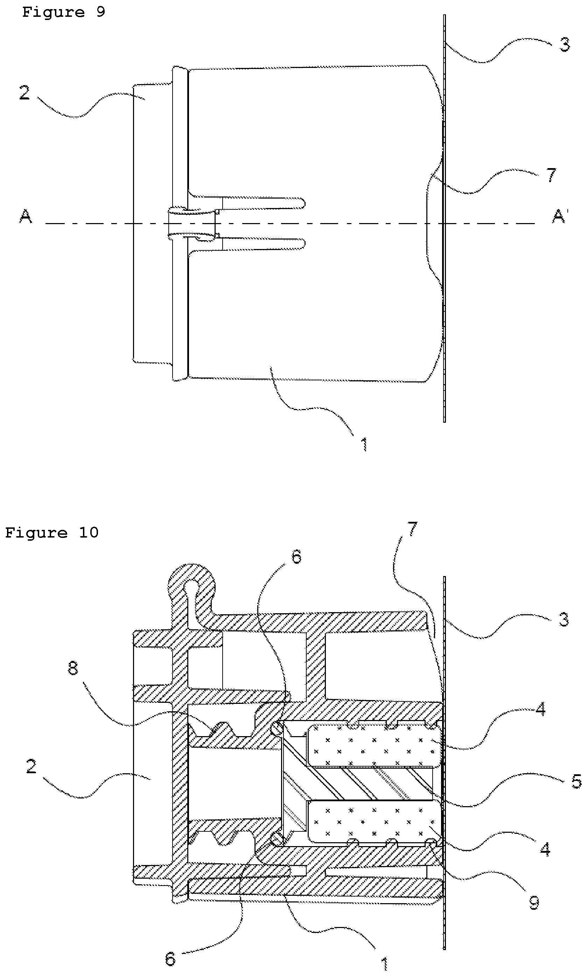

- Figure 9:: Top view of the product with the caps being closed,
- Figure 10:: Side A-A' section view of the product with the caps being closed,

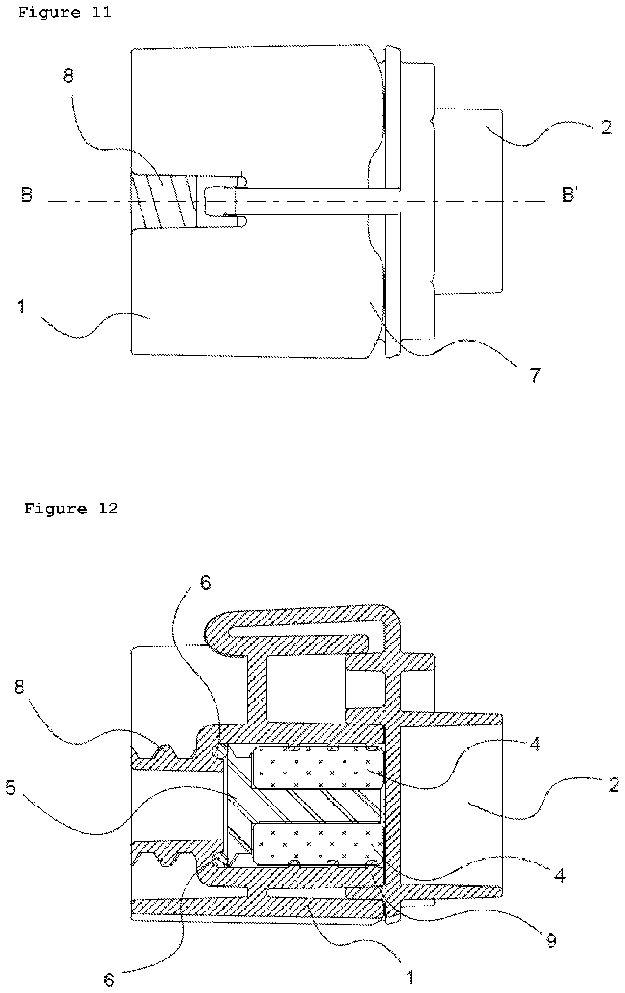

- Figure 11:: Top view of the product with the caps being open,
- Figure 12:: Side B-B' section view of the product with the caps being open,

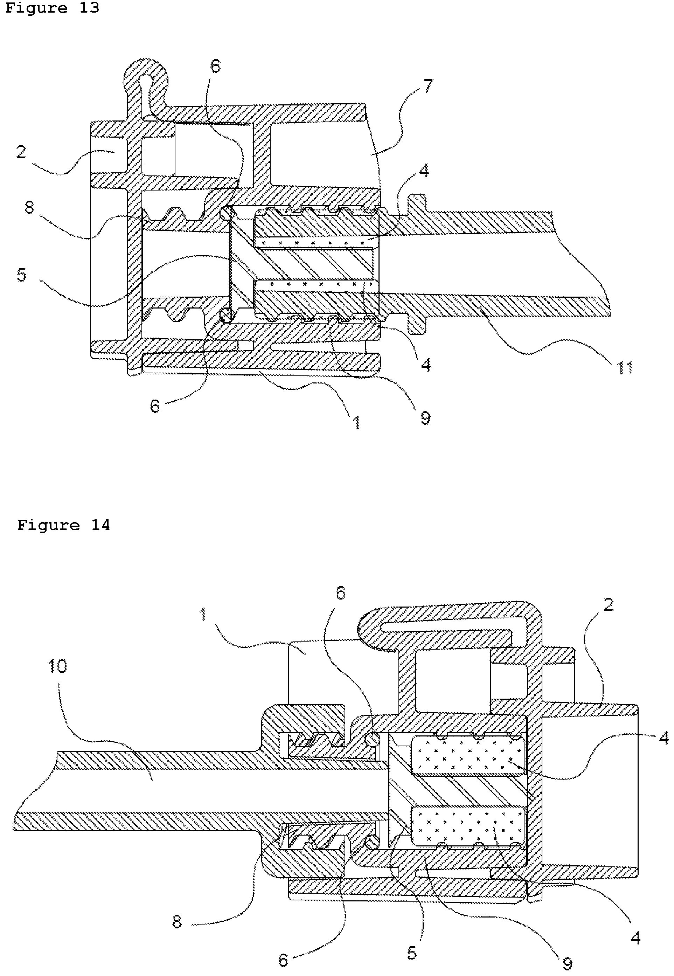

- Figure 13:: Side section view of the product with the female connection being inserted,
- Figure 14:: Side section view of the product with the made connection being inserted,

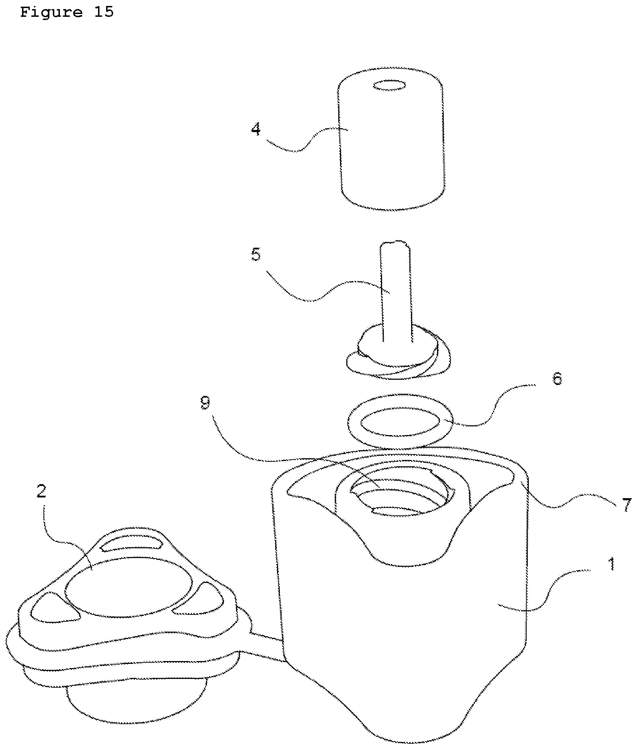

- Figure 15:: Perspective view of the product as exploded,

### Explanation of References in the Drawings

The parts in the drawings are enumerated and their explanations are given below:
- 1:: Body
- 2:: Cap
- 3:: Foil Cap
- 4:: Disinfectant Holder Member
- 5:: Piston
- 6:: Seal
- 7:: Curved Surface
- 8:: Slot suitable for the male connection
- 9:: Slot suitable for the female connection
- 10:: Male connection
- 11:: Female connection

### Description of the Invention

The disinfection cap compatible for multiple connections consists of the main parts such as body (1), cap (2), foil cap (3) adhered to the body, disinfectant holder member (4), piston (5) and seal (6) supporting the piston (5). While the body (1) forms a reference to the gripping style with its triangular form, thanks to its flat surface, it eliminates the areas where microbial settlement may occur. Two caps (2, 3) positioned mutually on the body (1) make the system closed. When the system is closed, the cap (2) protects the part where the female connection is provided, and the foil cap (3) protects the part where the male connection is provided. In cases where the cap (2), which is compatible with both open parts of the body (1), will be provided with a female connection (11), the foil cap (3) is inserted in its place by folding and protects the system from the external factors such as dust and particles.

The part where the male connection (10) will be made is differentiated as a curved surface (7), preventing the curved surface (7) from contacting the foil cap (3). It is ensured that the surface where the disinfectant holder member (4) is located is covered on the curved surface (7). By reducing the contact surface, the separation of the body (1) from the foil cap (3) is facilitated. The channels inside the body (1) form the compatible slots (8,9) with the male and female connections (10,11).

The piston (5) is located in the center of the system and is positioned in such a way that the disinfectant holder member (4) is compressed when the pressure comes from the male connection (10). The seal (6) is positioned adjacent to the piston (5) and prevents the movement of the piston (5) which may occur due to the increase in the pressure in the female connections (11), while it ensures sealing as the disinfectant solution, antibacterial solution, antifungal solution or isopropyl alcohol is spreading to the female connection (11) that comes out because of the pressure on the disinfectant holder member (4) coming from the piston (5) which moves by a thrust force on the male connections (10).

When the system is used for the female connection (11), the movement of the piston (5) compresses the disinfectant holder member (4) horizontally and vertically, and due to the pressure, the disinfectant solution, antibacterial solution, antifungal solution or isopropyl alcohol is spread to all surfaces of the female connection (11). When it is used for the male connection (10), the disinfectant holder member (4) remaining between the connector and the piston (5) is compressed only in the vertical direction. With the compression of the disinfectant holder member, the distribution of the disinfectant solution, antibacterial solution, antifungal solution or isopropyl alcohol provides a contact to the entire surface of the connection piece including inside and outside thereof. Furthermore, the fact that the foil cap (3) is connected each other in certain number of sets and produced in a batch structure enables the practitioner to store the desired number of products without affecting the closure of the system.

### Industrial Applicability of the Invention

As mentioned above, the disinfection cap which successfully solves the existing problems can be produced like the similar ones available in the market. Production of body and caps is provided with traditional production methods.

## Claims

1. Disinfection cap for needle-free medical valve connections, enabling female and male connection type on one body (1), **characterized in that** it comprises;
• a slot (8) suitable for male connection (10), enabling the male connection to be connected, and a slot (9) suitable for female connection (11), enabling the male connection to be connected, positioned at opposite ends on the same body (1),
• a cap (2) which closes one of the slots (8, 9) by making the system closed, and a foil cap (3) which closes the other by making the system closed,
• a cap (2) which is designed to be suitable for both open parts of the body (1) in order to cover both slots (8, 9) and which can be folded to two opposite ends at the connection point on the axis of the body (1), connected to the body (1) in order to close both slots (8, 9).

2. Disinfection cap according to claim 1, **characterized in that** the cap (2) protects the slot (9) suitable for the female connection when the system is closed and a foil cap (3) protecting the slot (8) suitable for the male connection.

3. Disinfection cap according to claim 2, **characterized in that** the cap (2) provides a system protection by closing the female connection slot (9) by folding on the body (1) during the male connection (10) and makes the system prepared by remaining closed on the male connection slot (8) during the female connection (11).

4. Disinfection cap according to claim 1, **characterized in that** it comprises a triangular body (1) having flat surfaces.

5. Disinfection cap according to claim 1, **characterized in that** it comprises a part where the male connection (10) is realized, differentiated in the form of a curved surface (7) to prevent contact with the foil cap (3).

6. Disinfection cap according to claim 1 or 5, **characterized in that** the foil cap (3) holds the disinfection caps together in the specified sets and is provided with cross-cuts in accordance with the triangular shape of the body (1) for easy tearing off.

7. Disinfection cap according to claim 1, **characterized in that** it comprises a piston (5) which is located in the center of the body (1), provides pressure to the disinfectant holder member (4) by moving with the male connection (10) entering the system and makes the system operable by staying stable with the female connection entering the system and by compressing the disinfectant holder member (4).

8. Disinfection cap according to claim 1 or 5, **characterized in that** it has a seal (6) which enables the disinfectant solution, antibacterial solution, antifungal solution or isopropyl alcohol released by pushing the piston (5) to come into contact in a limited area during the male connection (10) and prevents the movement of the piston (5) with the effect of the pressure during the female connection (11),

## Patentansprüche

1. Desinfektionskappe für nadelfreie medizinische Ventilanschlüsse, die an einem Gehäuse (1) sowohl einen weiblichen als auch einen männlichen Anschluss ermöglicht, **dadurch gekennzeichnet, dass** sie Folgendes umfasst:
• eine für einen Steckverbinder (10) geeignete Aufnahme (8), die das Anschließen des männliche Steckverbinders ermöglicht und an den gegenüberliegenden Enden desselben Gehäuses (1) angeordnet ist,
• eine Kappe (2), die einen der Schlitze (8, 9) verschließt, wodurch das System geschlossen wird, und eine Folienkappe (3), die den anderen verschließt, wodurch das System geschlossen wird,
• eine Abdeckung (2), die so gestaltet ist, dass sie auf beide Öffnungen des Gehäuses (1) passt, um beide Schlitze (8, 9) abzudecken, und die an der Verbindungsstelle auf der Achse des Gehäuses (1) zu zwei gegenüberliegenden Enden gefaltet werden kann, wobei sie mit dem Gehäuse (1) verbunden ist, um beide Schlitze (8, 9) zu verschließen.

2. Desinfektionskappe nach Anspruch 1, **dadurch gekennzeichnet, dass** die Kappe (2) den für den weiblichen Anschluss vorgesehenen Schlitz (9) schützt, wenn das System geschlossen ist, sowie eine Folienkappe (3), die den für den männlichen Anschluss vorgesehenen Schlitz (8) schützt.

3. Desinfektionskappe nach Anspruch 2, **dadurch gekennzeichnet, dass** die Kappe (2) einen Systemschutz bietet, indem sie den Anschlusssteckplatz (9) für den weiblichen Stecker durch Umklappen am Körper (1) während des Anschlusses des männlichen Steckers (10) verschließt, und das System bereitstellt, indem sie während des Anschlusses des weiblichen Steckers (11) auf dem Anschlusssteckplatz (8) für den männlichen Stecker geschlossen bleibt.

4. Desinfektionskappe nach Anspruch 1, **dadurch gekennzeichnet, dass** sie einen dreieckigen Körper (1) mit ebenen Flächen umfasst.

5. Desinfektionskappe nach Anspruch 1, **dadurch gekennzeichnet, dass** sie einen Abschnitt aufweist, an dem der männliche Steckanschluss (10) ausgebildet ist, der in Form einer gekrümmten Fläche (7) gestaltet ist, um einen Kontakt mit der Folienkappe (3) zu verhindern.

6. Desinfektionskappe gemäß Anspruch 1 oder 5, **dadurch gekennzeichnet, dass** die Folienkappe (3) die Desinfektionskappen in den vorgegebenen Sets zusammenhält und zum einfachen Abreißen mit Einschnitten versehen ist, die der dreieckigen Form des Körpers (1) entsprechen.

7. Desinfektionskappe nach Anspruch 1, **dadurch gekennzeichnet, dass** sie einen Kolben (5) umfasst, der sich in der Mitte des Gehäuses (1) befindet, durch seine Bewegung mit dem in das System eintretenden männlichen Anschluss (10) Druck auf das Desinfektionsmittel-Halteelement (4) ausübt und das System betriebsbereit macht, indem er bei in das System eintretendem weiblichen Anschluss stabil bleibt und das Desinfektionsmittel-Halteelement (4) zusammendrückt.

8. Desinfektionskappe nach Anspruch 1 oder 5, **dadurch gekennzeichnet, dass** sie eine Dichtung (6) aufweist, die es ermöglicht, dass die durch Drücken des Kolbens (5) freigesetzte Desinfektionslösung, antibakterielle Lösung, antimykotische Lösung oder Isopropylalkohol während des männlichen Anschlusses (10) in einem begrenzten Bereich in Kontakt kommt, und die Bewegung des Kolbens (5) unter dem Einfluss des Drucks während des weiblichen Anschlusses verhindert (11).

## Revendications

1. Bouchon de désinfection pour connexions de valves médicales sans aiguille, permettant un type de connexion femelle et mâle sur un seul corps (1), **caractérisé en ce qu'**il comprend ;
• une fente (8) adaptée à une connexion mâle (10), permettant le raccordement de la connexion mâle, et une fente (9) adaptée à une connexion femelle (11), permettant également le raccordement de la connexion mâle, positionnées aux extrémités opposées du même corps (1),
• un capuchon (2) qui ferme l'une des fentes (8, 9) en fermant le système, et un capuchon en feuille d'aluminium (3) qui ferme l'autre en fermant le système.
• un capuchon (2) qui est conçu pour convenir aux deux parties ouvertes du corps (1) afin de couvrir les deux fentes (8, 9) et qui peut être plié à deux extrémités opposées au point de connexion sur l'axe du corps {1}, connecté au corps (1) afin de fermer les deux fentes (8, 9).

2. Bouchon de désinfection selon la revendication 1, **caractérisé en ce que** le bouchon (2) protège la fente (9) adaptée à la connexion femelle lorsque le système est fermé et un bouchon en feuille (3) protège la fente (8) adaptée à la connexion mâle.

3. Bouchon de désinfection selon la revendication 2, **caractérisé en ce que** le bouchon (2) assure une protection du système en fermant la fente de connexion femelle (9) en se repliant sur le corps (1) pendant la connexion mâle (10) et prépare le système en restant fermé sur la fente de connexion mâle (8) pendant la connexion femelle (11).

4. Bouchon de désinfection selon la revendication 1, **caractérisé en ce qu'**il comprend un corps triangulaire (1) ayant des surfaces planes.

5. Capuchon de désinfection selon la revendication 1, **caractérisé en ce que** il comprend une partie où la connexion mâle ( 10 ) est réalisée, différenciée, sous la forme d'une surface incurvée ( 7 ) pour empêcher le contact avec le capuchon en feuille ( 3 ).

6. Bouchon de désinfection selon la revendication 1 ou 5, **caractérisé en ce que** le bouchon en aluminium (3) maintient les bouchons de désinfection ensemble dans les ensembles spécifiés et est muni de découpes transversales en accord avec la forme triangulaire du corps (1) pour un arrachage facile.

7. Bouchon de désinfection selon la revendication 1, **caractérisé en ce qu'**il comprend un piston ( 5 ) qui est situé au centre du corps (1 ), fournit une pression à l'élément porte-désinfectant (4) en se déplaçant avec le raccord mâle (10) entrant dans le système et rend le système opérationnel en restant stable avec le raccord femelle entrant dans le système et en comprimant l'élément porte-désinfectant (4).

8. Bouchon de désinfection selon la revendication 1 ou 5, **caractérisé en ce qu'**il comporte un joint (6) qui permet à la solution désinfectante, antibactérienne, antifongique ou à l'alcool isopropylique libérée par la poussée du piston (5) d'entrer en contact dans une zone limitée pendant la connexion mâle (10) et empêche le mouvement du piston (5) sous l'effet de la pression pendant la connexion femelle (11).
